# EUROPEAN PATENT APPLICATION

(11) **EP 3 489 960 A1**
(43) Date of publication of application: **29.05.2019**
(21) Application number: 17203595.8
(22) Date of filing: 24.11.2017
(51) Int. Cl.: G16H 20/00, G16H 50/70

(54) **AN APPARATUS AND METHOD FOR CARE PLAN GENERATION**

(71) Applicant: Koninklijke Philips N.V., 5656 AE Eindhoven (NL)
(72) Inventor: ZHU, Jane, 5656 AE Eindhoven (NL); CHAN, Tak Ming, 5656 AE Eindhoven (NL); ZHENG, Chuan, 5656 AE Eindhoven (NL)
(74) Representative: de Haan, Poul Erik

(57) **Abstract**

There is provided a computer-implemented method for generating a care plan for a subject. A plurality of care plans stored in one or more databases are accessed (202). The plurality of care plans are grouped into a plurality of care plan categories based on a similarity measure between one or more attributes of the plurality of care plans. Each of the plurality of care plans is associated with a reference subject. A care plan category for the subject is identified from the plurality of care plan categories using a trained classifier (204). A care plan for the subject is generated (206) based on the one or more care plans that are grouped into the identified care plan category, by maximizing a similarity measure between one or more attributes of the subject and one or more corresponding attributes of the reference subjects.

## Description

### FIELD OF THE INVENTION

The present disclosure relates to an apparatus and method for generating a care plan for a subject.

### BACKGROUND OF THE INVENTION

In post-hospital (post-discharge) management systems currently available, physicians can create a personalized care plan for each patient who joins the post-discharge management program. For each patient, a care plan may include a number of separate sub-plans. For example, for a typical coronary artery disease (CAD) patient, a care plan may include a medication plan, a home monitoring plan, an education and coaching plan, and a follow-up plan, etc. It is time-saving for the physician if they can quickly reach an ideal or intended care plan from a recommended one provided by an automated system, while being able to view the reasoning of the recommendation for reference purposes.

In some currently available solutions, pre-defined protocols, such as possible care elements or possible attributes of care elements in a care plan, can be shown in a list and can be selected by physicians to form an initial plan template. However, in many cases, for certain types of patients the protocols cannot be pre-defined and in some cases the pre-defined protocols cannot cover all possible care plans for a large number of subjects.

Moreover, in some currently available solutions, users or medical personnel (e.g. physicians) are allowed to save existing care plans individually as templates in a database. The saved templates can be searched by users and selected as an initial care plan template for a new patient. However, in this case, there are too many templates in the database which may make it difficult for a user to select a suitable template.

### SUMMARY OF THE INVENTION

As noted above, there are a number of disadvantages associated with the currently available post-hospital management systems for selecting a care plan for a subject. For example, the management systems that employ pre-defined protocols cannot fully cover all possible care plans for a large number of subjects. As another example, the users of management systems that use historical care plans as templates may find it difficult to navigate through the plurality of care plan templates for a match with a new subject. Moreover, it is difficult and time-consuming for users to manually set up a detailed executable care plan using these management systems, especially when the parameters, i.e. attributes of the care elements in the care plans, available for selection are not as close as possible to the final ideal care plan so a user may have to either select a less accurate parameter or not select a parameter at all.

It would therefore be advantageous to provide an improved method for generating a care plan for a subject.

To better address one or more of the concerns mentioned earlier, in a first aspect, a computer-implemented method for generating a care plan for a subject is provided. The method comprises accessing a plurality of care plans stored in one or more databases, wherein the plurality of care plans are grouped into a plurality of care plan categories based on a similarity measure between one or more attributes of the plurality of care plans, and wherein each of the plurality of care plans is associated with a reference subject. The method also comprises identifying a care plan category for the subject from the plurality of care plan categories using a trained classifier and generating a care plan for the subject based on the one or more care plans stored in the one or more databases that are grouped into the identified care plan category, by maximizing a similarity measure between one or more attributes of the subject and one or more corresponding attributes of the reference subjects.

In some embodiments, the similarity measure between one or more attributes of the plurality of care plans may be based on a number of attributes the one or more care plans have in common and/or that differ by less than a threshold. In these embodiments, two care plans with a similarity measure exceeding a predetermined minimum similarity threshold may be grouped in the same care plan category. In some embodiments, the similarity measure between the one or more attributes of the subject and the one or more corresponding attributes of the reference subjects may be based on the number of attributes the subject has in common with the reference subject and/or that differ by less than a threshold.

In some embodiments, at predetermined time intervals, the plurality of care plans may be regrouped into an updated plurality of care plan categories and the classifier may be retrained.

In some embodiments, the method may further comprise outputting the identified care plan category and the one or more care plans that are grouped into the identified care plan category. In some embodiments, a plurality of care plans may be grouped into the identified care plan category, and the method may further comprise outputting the similarity measures between the care plans that are grouped into the identified care plan category.

In some embodiments, each reference subject may be labelled with a label that identifies the care plan category comprising the care plan for the reference subject. In these embodiments, the method may further comprise: filtering reference subjects from the one or more databases based on the identified care plan category to select candidate reference subjects. The candidate reference subjects may comprise reference subjects with a label identifying the reference subjects as having care plans grouped into the identified care plan category. Moreover, in these embodiments, generating a care plan for the subject may comprise: generating a care plan for the subject, based on the one or more care plans stored in the one or more databases that are grouped into the identified care plan category, by maximizing a similarity measure between one or more attributes of the subject and one or more corresponding attributes of the candidate reference subjects.

In some embodiments, the method may further comprise grouping the care plans associated with the candidate reference subjects into a plurality of care plan sub-categories based on a similarity measure between the care plans associated with the candidate reference subjects. In these embodiments, the method may further comprise training a further classifier to identify a care plan sub-category for the subject from the plurality of care plan sub-categories.

In some embodiments, the method may further comprise compiling an ordered list of the care plans of the identified care plan category, wherein the list is ordered based on a similarity measure between one or more attributes of the subject and one or more corresponding attributes of each of the reference subjects. In these embodiments, the higher the similarity measure between the one or more attributes of the subject and one or more attributes of a reference subject, the higher the care plan associated with the reference subject may be in the list.

In a second aspect, there is provided a computer program product comprising a computer readable medium, the computer readable medium having computer readable code embodied therein, the computer readable code being configured such that, on execution by a suitable computer or processor, the computer or processor is caused to perform the method described above.

In a third aspect, there is provided an apparatus for generating a care plan for a subject. The apparatus comprises a processor configured to access a plurality of care plans stored in one or more databases, wherein the plurality of care plans are grouped into a plurality of care plan categories based on a similarity measure between one or more attributes of the plurality of care plans, and wherein each of the plurality of care plans is associated with a reference subject. The processor is further configured to identify a care plan category for the subject from the plurality of care plan categories using a trained classifier and generate a care plan for the subject based on the one or more care plans stored in the one or more databases that are grouped into the identified care plan category, by maximizing a similarity measure between one or more attributes of the subject and one or more corresponding attributes of the reference subjects.

According to the aspects and embodiments described above, the limitations of existing techniques are addressed. In particular, the above-described aspects and embodiments enable an automatic recommendation of a care plan to be performed. The automatic recommendation takes into account similar historical care plans and the similarity between one or more attributes of the subject and one or more corresponding attributes of a number of reference subjects. In this way, prediction errors can be reduced. Moreover, the method does not require the use of predefined protocols or templates. This improves the efficiency and accuracy for care plan generation. There is thus provided an improved method and apparatus for generating a care plan for a subject.

These and other aspects of the disclosure will be apparent from and elucidated with reference to the embodiment(s) described hereinafter.

### BRIEF DESCRIPTION OF THE DRAWINGS

For a better understanding of the embodiments, and to show more clearly how they may be carried into effect, reference will now be made, by way of example only, to the accompanying drawings, in which:
Fig. 1 is a block diagram of an apparatus for generating a care plan for a subject according to an embodiment; and
Fig. 2 illustrates a method for generating a care plan for a subject according to an embodiment.

### DETAILED DESCRIPTION OF EMBODIMENTS

As noted above, there is provided an improved apparatus and a method of operating the same which addresses the existing problems.

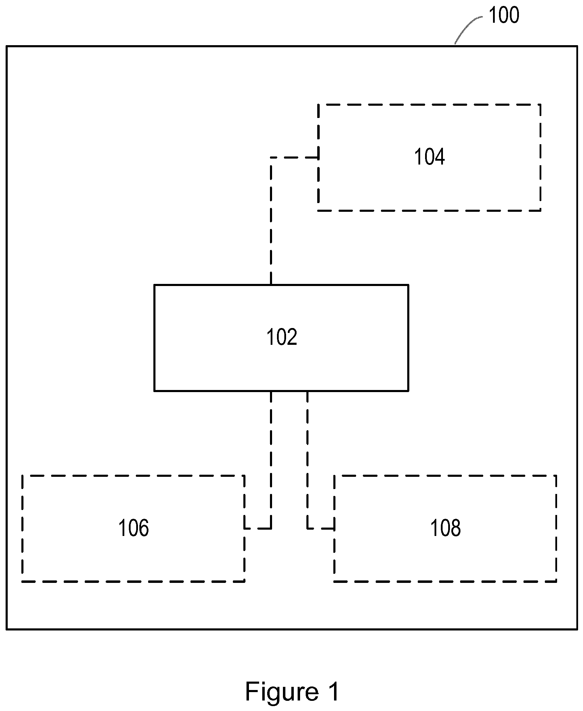

**Fig. 1** shows a block diagram of an apparatus 100 according to an embodiment, which can be used for generating a care plan for a subject. A care plan can comprise a plurality of care elements with attributes presented in the same information structure. Each care element may be associated with a specific class and one or more attributes. A care plan may contain instructions to a user (e.g. a medical practitioner) regarding a subject's health condition, for example, a timetable for doing physical exercise or instructions for taking medication. In some embodiments, the one or more attributes of the plurality of care plans can comprise one or more clinical attributes of the plurality of care plans.

For example, a care plan may include a medication plan including a list of medication. In this example, each medication in the list is a care element associated with the "medication" class. Also, in this example, each medication in the list is associated with one or more attributes, e.g. a medicine name, a medicine dose, a medicine frequency, a medicine effect duration period, a time of day for taking the medicine (e.g. morning, evening), a chemical structure of a medicine, a pharmacology of a medicine, a manufacturer of a medicine, etc. A care plan which only comprises care elements with attributes that are highly clinically-relevant is referred to as a "high-level care plan", and a care plan which comprises care elements with the necessary attributes to be executed by a subject is referred to as a "detailed care plan".

A care plan may comprise other types of plans (i.e. plans associated with other classes), such as a nutrition care plan, an exercise care plan, a mental health care plan, a home monitoring plan, an education and/or coaching plan, a follow-up plan. The care elements contained in these types of plans are respectively associated with classes such as "nutrition care", "exercise", "mental health", "home monitoring", "education and/or coaching", and "follow-up".

As illustrated in Fig. 1, the apparatus comprises a processor 102 that controls the operation of the apparatus 100 and that can implement the method described herein. The processor 102 can comprise one or more processors, processing units, multi-core processor or modules that are configured or programmed to control the apparatus 100 in the manner described herein. In particular implementations, the processor 102 can comprise a plurality of software and/or hardware modules that are each configured to perform, or are for performing, individual or multiple steps of the method described herein.

Briefly, the processor 102 is configured to access a plurality of care plans stored in one or more databases. The plurality of care plans are grouped into a plurality of care plan categories based on a similarity measure between one or more attributes of the plurality of care plans and each of the plurality of care plans is associated with a reference subject. The processor 102 is also configured to identify a care plan category for the subject from the plurality of care plan categories using a trained classifier. The processor 102 is further configured to generate a care plan for the subject based on the one or more care plans stored in the one or more databases that are grouped into the identified care plan category, by maximizing a similarity measure between one or more attributes of the subject and one or more corresponding attributes of the reference subjects.

The one or more attributes of the subject referred to herein may, for example, comprise any one or more of: clinical attributes of the subject, demographic attributes of the subject, and administrative attributes of the subject. For example, the one or more attributes of the subject may comprise a diagnosis of the subject (which is an example of a clinical attribute), an age of the subject (which is an example of a demographic attribute), and/or a physician of the subject (which is an example of an administrative attribute).

In some embodiments, the apparatus 100 may further comprise at least one user interface 104. Alternative or in addition, at least one user interface 104 may be external to (i.e. separate to or remote from) the apparatus 100. For example, at least one user interface 104 may be part of another device. A user interface 104 may be for use in providing a user of the apparatus 100 with information resulting from the method described herein. For example, the processor 102 may be configured to control one or more user interfaces 104 to render (or output or display) the generated care plan(s) for the subject. Alternative or in addition, a user interface 104 may be configured to receive a user input. For example, a user interface 104 may allow a user of the apparatus 100 to manually enter instructions, data, or information. In these embodiments, the processor 102 may be configured to acquire the user input from one or more user interfaces 104.

A user interface 104 may be any user interface that enables the rendering (or output or display) of information to a user of the apparatus 100. Alternatively or in addition, a user interface 104 may be any user interface that enables a user of the apparatus 100 to provide a user input, interact with and/or control the apparatus 100. For example, the user interface 104 may comprise one or more switches, one or more buttons, a keypad, a keyboard, a touch screen or an application (for example, on a tablet or smartphone), a display screen, a graphical user interface (GUI) or other visual rendering component, one or more speakers, one or more microphones or any other audio component, one or more lights, a component for providing tactile feedback (e.g. a vibration function), or any other user interface, or combination of user interfaces.

In some embodiments, the apparatus 100 may comprise a memory 106. Alternatively or in addition, one or more memories 106 may be external to (i.e. separate to or remote from) the apparatus 100. For example, one or more memories 106 may be part of another device. A memory 106 can be configured to store program code that can be executed by the processor 102 to perform the method described herein. A memory can be used to store information, data, signals and measurements acquired or made by the processor 102 of the apparatus 100. For example, a memory 106 may be used to store (for example, in a local file) a plurality of care plans and one or more attributes of the plurality of care plans. The processor 102 may be configured to control a memory 106 to store the plurality of care plans and one or more attributes of the plurality of care plans.

In some embodiments, the apparatus 100 may comprise a communications interface (or circuitry) 108 for enabling the apparatus 100 to communicate with any interfaces, memories and/or devices that are internal or external to the apparatus 100. The communications interface 108 may communicate with any interfaces, memories and/or devices wirelessly or via a wired connection. For example, the communications interface 108 may communicate with one or more user interfaces 104 wirelessly or via a wired connection. Similarly, the communications interface 108 may communicate with the one or more memories 106 wirelessly or via a wired connection.

It will be appreciated that Fig. 1 only shows the components required to illustrate an aspect of the apparatus 100 and, in a practical implementation, the apparatus 100 may comprise alternative or additional components to those shown.

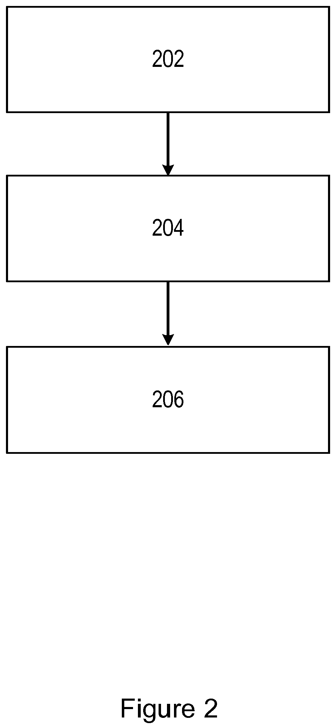

**Fig. 2** illustrates a computer-implemented method for generating a care plan for a subject according to an embodiment. The illustrated method can generally be performed by or under the control of processor 102 of the apparatus 100.

With reference to Fig. 2, at block 202, a plurality of care plans stored in one or more databases is accessed. More specifically, the plurality of care plans is accessed by the processor 102 of the apparatus 100. In some embodiments, the plurality of care plans may be accessed from one or more databases in a memory 106, which may be a memory of the apparatus 100 or a memory external to the apparatus 100. Each of the plurality of care plans is associated with a reference subject. A reference subject may, for example, be a patient who was admitted to a clinical environment, such as a hospital.

In addition, the plurality of care plans are grouped into a plurality of care plan categories based on a similarity measure between one or more attributes of the plurality of care plans. This grouping may be referred to as "clustering" and may be performed using a clustering algorithm. A person skilled in the art will be aware of various clustering algorithms that may be used to group the plurality of care plans into a plurality of care plan categories. However, in some embodiments, for example, the Tanimoto Coefficient may be used, as part of a clustering algorithm, for the grouping of the plurality of care plans. In some embodiments, the plurality of care plans that are grouped into a plurality of care plan categories may be "high-level care plans", which only comprise care elements that are highly clinically-relevant.

As mentioned above, a care plan can comprise a plurality of care elements with attributes presented in the same information structure. Each care element may be associated with a specific class and one or more attributes. In some embodiments, the similarity measure between one or more attributes of the plurality of care plans may be based on a number of attributes the one or more care plans have in common and/or that differ by less than a threshold. In some embodiments, at block 202 of Fig. 2 or before performing the computer-implemented method of Fig. 2, two care plans with a similarity measure exceeding a predetermined minimum similarity threshold may be grouped in the same care plan category.

For example, the similarity measure between one care plan and another care plan in a plurality of care plans may be represented by a number indicative of the number of attributes that the two care plans have in common, and the predetermined minimum similarity threshold may be an integer number (such as 5 or any other integer number). Thus, in this example, two care plans are grouped into the same care plan category where the two care plans have at least the integer number of attributes in common. In some embodiments, grouping of the plurality of care plans into a plurality of care plan categories may comprise, for each of the plurality of care plans, determining the similarity measure between the care plan and each of the rest of the plurality of care plans and then comparing the determined similarity measures against the predetermined minimum similarity threshold. In an example where the predetermined minimum similarity threshold is an integer number of 5, if the similarity measure between a first care plan P₁ and a second care plan P₂ is 6, then the processor 102 groups the first care plan P₁ and the second care plan P₂ in the same care plan category. In the same example, if a similarity measure between the first care plan P₁ and a third care plan P₃ is 3, then the processor 102 does not group the first care plan P₁ or the second care plan P₂ with the third care plan P₃ in the same care plan category. Thus, the third care plan P₃ in this example is grouped in a different care plan category to the first care plan P1 and the second care plan P2.

Returning back to Fig. 2, at block 204, a care plan category for the subject is identified from the plurality of care plan categories, using a trained classifier. In some embodiments, the classifier may be trained based on the plurality of care plans stored in the one or more databases and their respective care plan categories, so as to allow prediction of a care plan category for a new subject. In some embodiments, the subject may be assigned with a label that is indicative of the identified care plan category for the subject. For example, in some embodiments, depending on the outcome at block 204 of Fig. 2, the subject may be assigned with a label which indicates that their identified care plan category is related to at least one of: a medication, a home measurement (e.g. a blood pressure measurement), and a follow-up procedure (e.g. a time period to follow-up with the subject by medical personnel).

Although not illustrated in Fig. 2, in some embodiments, the plurality of care plans may be regrouped into an updated plurality of care plan categories. In these embodiments, the classifier may be retrained. The regrouping of the plurality of care plans and/or the retraining of the classifier may be performed at predetermined time intervals, e.g. weekly, or may be triggered by a new event such as an entry of a new care plan in the one or more databases.

Also, although not illustrated in Fig. 2, the computer-implemented method in some embodiments may further comprise outputting the identified care plan category and the one or more care plans that are grouped into the identified care plan category. In these embodiments, the one or more care plans may comprise visualized information. For example, the one or more care plans that are grouped into the identified care plan category may be presented visually via a user interface 104 so as to provide medical personnel (e.g. a physician) with an overview of the (similar) care plans on which the generation of the care plan for the subject is based. This may help medical personnel gain a better clinical understanding of the (similar) care plans. In some embodiments, the computer-implemented method may further comprise outputting the similarity measures between the care plans that are grouped into the identified care plan category.

Returning back to Fig. 2, at block 206, a care plan for the subject is generated. This generating step is performed based on the one or more care plans stored in the one or more databases, specifically from the one or more care plans that are grouped into the care plan category that is identified at block 204 of Fig. 2, and by maximizing a similarity measure between one or more attributes of the subject and one or more corresponding attributes of the reference subjects. In some embodiments, the generation of the care plan for the subject may comprise including attributes in the generated care plan based on attributes of the care plans that are grouped in the care plan category identified at block 204 of Fig. 2 and at the same time maximizing a similarity measure between one or more attributes of the subject and one or more corresponding attributes of the reference subjects.

As an example, if at block 204 of Fig. 2 a care plan category relating to a blood pressure measurement is identified, then at block 206 of Fig. 2, the generation of a care plan for the subject may comprise including in the care plan for the subject attributes of the care plans relating to a blood pressure measurement of the reference subject(s) who have similar attributes (e.g. diagnosis) to the subject. Specifically, in this example, if the care plans of the reference subject(s) who have similar attributes (e.g. diagnosed with hyper tension) to those of the subject comprise an attribute of "3 times per day" relating to a blood pressure measurement, the processor 102 may generate the care plan for the subject by including the attribute "3 times per day" with regard to a blood pressure measurement in the care plan. It will be appreciated that a similar process may be applied to other care plan categories and other attributes in generating a care plan for the subject.

In some embodiments, the similarity measure between the one or more attributes of the subject and the one or more corresponding attributes of the reference subjects may be based on the number of attributes the subject has in common with the reference subject and/or that differ by less than a threshold. As mentioned above, the one or more attributes of the subject may comprise any one or more of: clinical attributes of the subject (e.g. a diagnosis), demographic attributes of the subject (e.g. an age), and administrative attributes of the subject. Therefore, in an example, if the subject has the same age as a reference subject this may be indicated by an increased value in the similarity measure between the one or more attributes of the subject and the one or more attributes of the reference subject. For example, a similarity measure between the one or more attributes of the subject and one or more corresponding attributes of a reference subject may be represented by a value which indicates the number of attributes the subject has in common with the reference subject, e.g. a value of "4" indicating that the subject has four attributes in common with the reference subject.

The computer-implemented method in some embodiments may further comprise compiling an ordered list of the care plans of the care plan category identified at block 204 of Fig. 2 based on a similarity measure between the one or more attributes of the subject and one or more corresponding attributes of each of the reference subjects. In some cases, the higher the similarity measure between the one or more attributes of the subject and one or more attributes of a reference subject, the higher the care plan associated with the reference subject is in the list (or the higher the priority is that is assigned to the care plan associated with the reference subject). For example, a care plan associated with a reference subject who has a similarity measure of "3" with the subject in terms of the number of common attributes may be assigned a higher priority than a care plan associated with a reference subject who has a similarity measure of "2" with the subject in terms of the number of common attributes. This may be output via a user interface 104 of the apparatus 100 so as to provide medical personnel (e.g. a physician) with a visualized overview of the detailed care plans that are relevant to the subject.

In some embodiments, each reference subject may be labelled with a label that identifies the care plan category comprising the care plan for the reference subject. In these embodiments, the computer-implemented method may further comprise filtering reference subjects from one or more databases based on the care plan category identified at block 204 of Fig. 2 in order to select candidate reference subjects. The candidate reference subjects may comprise reference subjects with a label identifying the reference subjects as having care plans grouped into the identified care plan category. In these embodiments, generating a care plan for the subject at block 204 of Fig. 2 may comprise generating a care plan for the subject based on the one or more care plans stored in the one or more databases that are grouped into the identified care plan category, by maximizing a similarity measure between one or more attributes of the subject and one or more corresponding attributes of the candidate reference subjects. Accordingly, in these embodiments, the generation of a care plan for the subject only takes into account attributes of reference subjects whose care plans belong to the care plan category identified at block 204 of Fig. 2.

In some of the embodiments involving candidate reference subjects, the care plans associated with the candidate reference subjects may be grouped into a plurality of care plan sub-categories based on a similarity measure between the care plans associated with the candidate reference subjects. The grouping of the care plans into care plan sub-categories may be based on a similarity measure between one or more attributes of the plurality of care plans. In these embodiments, the computer-implemented method may further comprise training a further classifier to identify a care plan sub-category for the subject from the plurality of care plan sub-categories.

Although not illustrated in Fig. 2, the computer-implemented method may comprise outputting the care plan for the subject that is generated at block 206 of Fig. 2. In some of these embodiments, reasoning for the generation of the care plan may also be output along with the generated care plan. The reasoning may include, for example, at least one of: a visualized explanation of the steps taken by the processor 102 to generate the care plan, and statistical information relating to one or more attributes included in the generated care plan (e.g. "measuring blood pressure three times per day is included in the care plan for the subject because 82% of historical subjects who are diagnosed with hypertension and who live in Beijing have the same instruction in their care plans").

There is thus provided an improved method and apparatus for generating a care plan for a subject, which overcomes the existing problems.

There is also provided a computer program product comprising a computer readable medium, the computer readable medium having computer readable code embodied therein, the computer readable code being configured such that, on execution by a suitable computer or processor, the computer or processor is caused to perform the method or methods described herein. Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method for generating a care plan for a subject, the method comprising:
accessing (202) a plurality of care plans stored in one or more databases, wherein the plurality of care plans are grouped into a plurality of care plan categories based on a similarity measure between one or more attributes of the plurality of care plans, and wherein each of the plurality of care plans is associated with a reference subject;
identifying (204) a care plan category for the subject from the plurality of care plan categories using a trained classifier; and
generating (206) a care plan for the subject based on the one or more care plans stored in the one or more databases that are grouped into the identified care plan category, by maximizing a similarity measure between one or more attributes of the subject and one or more corresponding attributes of the reference subjects.

2. A computer-implemented method according to claim 1, wherein the similarity measure between one or more attributes of the plurality of care plans is based on a number of attributes the one or more care plans have in common and/or that differ by less than a threshold.

3. A computer-implemented method according to claim 2, wherein two care plans with a similarity measure exceeding a predetermined minimum similarity threshold are grouped in the same care plan category.

4. A computer-implemented method according to any preceding claim, wherein the similarity measure between the one or more attributes of the subject and the one or more corresponding attributes of the reference subjects is based on the number of attributes the subject has in common with the reference subject and/or that differ by less than a threshold.

5. A computer-implemented method according to any of the preceding claims, wherein, at predetermined time intervals, the plurality of care plans are regrouped into an updated plurality of care plan categories and the classifier is retrained.

6. A computer-implemented method according to any of the preceding claims, the method further comprising:
outputting the identified care plan category and the one or more care plans that are grouped into the identified care plan category.

7. A computer-implemented method according to any preceding claim, wherein a plurality of care plans are grouped into the identified care plan category, and wherein the method further comprises:
outputting the similarity measures between the care plans that are grouped into the identified care plan category.

8. A computer-implemented method according to any of the preceding claims, where each reference subject is labelled with a label that identifies the care plan category comprising the care plan for the reference subject.

9. A computer-implemented method according to claim 8, the method further comprising:
filtering reference subjects from the one or more databases based on the identified care plan category to select candidate reference subjects, wherein the candidate reference subjects comprise reference subjects with a label identifying the reference subjects as having care plans grouped into the identified care plan category; and
wherein generating a care plan for the subject comprises:
generating a care plan for the subject, based on the one or more care plans stored in the one or more databases that are grouped into the identified care plan category, by maximizing a similarity measure between one or more attributes of the subject and one or more corresponding attributes of the candidate reference subjects.

10. A computer-implemented method according claim 9, further comprising:
grouping the care plans associated with the candidate reference subjects into a plurality of care plan sub-categories based on a similarity measure between the care plans associated with the candidate reference subjects.

11. A computer-implemented method according to claim 10, further comprising:
training a further classifier to identify a care plan sub-category for the subject from the plurality of care plan sub-categories.

12. A computer-implemented method according to any of the preceding claims, further comprising:
compiling an ordered list of the care plans of the identified care plan category, wherein the list is ordered based on a similarity measure between one or more attributes of the subject and one or more corresponding attributes of each of the reference subjects.

13. A computer-implemented method according to claim 12, wherein the higher the similarity measure between the one or more attributes of the subject and one or more attributes of a reference subject, the higher the care plan associated with the reference subject is in the list.

14. A computer program product comprising a computer readable medium, the computer readable medium having computer readable code embodied therein, the computer readable code being configured such that, on execution by a suitable computer or processor, the computer or processor is caused to perform the method as claimed in any of claims 1 to 13.

15. An apparatus (100) for generating a care plan for a subject, the apparatus comprising a processor (102) configured to:
access a plurality of care plans stored in one or more databases, wherein the plurality of care plans are grouped into a plurality of care plan categories based on a similarity measure between one or more attributes of the plurality of care plans, and wherein each of the plurality of care plans is associated with a reference subject;
identify a care plan category for the subject from the plurality of care plan categories using a trained classifier; and
generate a care plan for the subject based on the one or more care plans stored in the one or more databases that are grouped into the identified care plan category, by maximizing a similarity measure between one or more attributes of the subject and one or more corresponding attributes of the reference subjects.
